# EUROPEAN PATENT SPECIFICATION

(11) **EP 3 871 659 B1**
(45) Date of publication and mention of the grant of the patent: **02.04.2025**
(21) Application number: 19808626.6
(22) Date of filing: 22.10.2019
(51) Int. Cl.: A61K 9/00, A61K 9/70, A61K 31/522, A61K 31/198, A61K 31/4045, A61K 31/405

(54) **TRANSDERMAL PATCHES FOR USE IN AURICULOTHERAPY**
TRANSDERMALPFLASTER ZUR VERWENDUNG IN DER AURICULOTHERAPIE
PANSEMENTS TRANSDERMIQUES POUR L'AURICULOTHÉRAPIE

(30) Priority: 25.10.2018 EP 18382756
(43) Date of publication of application: 01.09.2021
(73) Proprietor: QM Health Care & Nutrition, S.L., 08023 Barcelona (ES)
(72) Inventor: NUÑEZ ZANCAS, Maria Luisa, 08349 CABRERA DE MAR (BARCELONA) (ES); CORTADELLAS RIBO, Blanca, 08017 BARCELONA (ES)
(74) Representative: Durán-Corretjer, S.L.P.
(86) International application number: PCT/ES2019/070715
(87) International publication number: WO 2020/084182

(56) References cited:
- GB-A- 2 438 926
- US-A1- 2015 010 619
- US-A1- 2018 243 251

## Description

The present invention is in the field of non-conventional medical treatments and particularly it refers to a transdermal patch for use in auriculotherapy comprising at least one active ingredient for the regulation of neurotransmitters for the treatment of physiological, neurological and neurohormonal disorders.

Western medicine has employed a traditional approach for the treatment of psychomental diseases based upon psycho-pharmaceuticals administered to the patient via the conventional oral intake. Some of the most widely used psycho-pharmaceuticals for the treatment of mood disorders, such as depression or anxiety, are based on the action of amino acids performing as neurotransmitters and thus regulating the nervous system.

On the other hand, presently it is known that other factors such as the nutritional and diet environment of the patient play a fundamental role in the prevention or treatment of several mental disorders, far more than what has been traditionally acknowledged. Different substances present in the human brain acting as neurotransmitters are known to perform decisive effects on a person's mood. Namely dopamine and norepinephrine both act as natural energetics; gamma-amino-butyric acid (GABA) is deemed a natural sedative, and endorphins and serotonin are considered stabilizers of the mood.

Serotonin, popularly known as the hormone of happiness, is produced by the transformation of the essential amino acid tryptophan, and acts as a neurotransmitter in the brain. Serotonin is synthesized within the serotoninergic neurons, mainly at the nerve endings, from tryptophan present in the blood in two stages: (a) hydroxylation through tryptophan hydroxylase into 5-hydroxytryptophan (5-HTP), and (b) the oxidative decarboxylation of 5-HTP into 5-hydroxytryptamine (serotonin).

In view of the above, serotonin synthesis is determined by the concentration of tryptophan available in the blood. As it is an essential amino acid, that is, not synthesized by the organism, tryptophan must be incorporated through the diet. However, the levels of tryptophan from ingested food are usually low and its concentration can be affected or reduced by stress and by the fact that it is also used as a precursor for the synthesis of vitamin B3 or nicotinic acid.

Therefore, there are only two mechanisms to increase the levels of serotonin in the body: i) by supplying serotonin precursors such as L-Tryptophan or the intermediate metabolite 5-HTP, or ii) by inhibiting serotonin reuptake, that is, preventing it from being reused by neurons with other purposes and thus increasing its availability.

Low serotonin levels have been associated with a number of psychiatric disorders such as anxiety and depression.

Antidepressants are among the most common drugs acting upon serotonin levels which, according to their action mechanism, can be differently classified as selective amine reuptake inhibitors (SSRIs), for example paroxetine chlorhydrate, non-selective amine reuptake inhibitors (NA/DA/5-HT), selective irreversible monoamine oxidase inhibitors, and non-selective and irreversible monoamine oxidase inhibitors (MAOls).

Despite the widespread use of said drugs, being paroxetine-chlorhydrate (ISRS) the second most purchased medicament worldwide employed to stimulate serotonin, prolonged use of such drugs results in a great number of side effects, such as headaches, vertigo, somnolence, dry mouth, loss of appetite, diaphoresis, insomnia, nausea, neuromuscular weakness, constipation and diarrhea. Furthermore, long term use of paroxetine chlorydrate causes high use dependency.

Another widely used antidepressant is selegiline (MAOI) which is prescribed for the treatment of early stages of Parkinson's disease, depression and senile dementia. This drug can be found in the market with different formulations, a transdermal patch among them, which can be applied on different parts of the body, such as the upper chest, the back, the thigh, or the arm, among others. However, among the many side effects associated to the use of such seligiline patches, skin reddening, difficulty for falling or staying asleep, diarrhea, heartburn, mouth dryness sensation, loss of weight, rash, severe headaches, faster than normal or irregular heart rate or with palpitations, chest pain, neck stiffness or pain, nausea, vomits, swelling, confusion, pupillary enlargement and higher sensibility to the light, can be found.

On the other hand, dopamine is a catecholamine acting as a neurotransmitter in the central nervous system, and it is also considered the neurohormone primarily involved in prolactine liberation inhibition.

The functions of dopamine in the brain are varied, regulating behavior and mood to start with, as well as motivation and reward, sleep, cognition or learning, and even controlling motor activity, among others.

Stimulant drugs, such as amphetamines and cocaine, achieve their pleasure effect altering the dopamine concentration in the nucleus accumbens. The nucleus accumbens is a small aggregate of cells found in the anterior brain connected to the amygdala and the limbic system. The nucleus accumbens is regarded as the brain's center of pleasure, as it contains most of the body's stored dopamine, being also sensitive to other pleasure neurotransmitters such as serotonin and endorphins.

Recent studies reveal that certain individuals' addictive tendencies towards psychosubstances might be related to low dopamine production, and that such tendency to consume addictive substances is an attempt of the individual to compensate the irregular levels of dopamine in their organism.

Conventionally, allopathic medicine uses different drugs for the treatment of dopamine dysfunctions, such as for instance, anticholinergics, dopaminergic agonists, receptor antagonists and blockers (including many anti-psychotics among them), monoamine oxidase B inhibitors, and catechol-O-methyl transferase (COMTs) inhibitors, among others.

For instance, rotigotine is a medicine which belongs to the group of the so called dopamine agonists, that is, those targeting post-sinaptic D2 dopaminergic receptors, and mainly used to control symptoms of Parkinson's disease, as well as pain resulting from the Willis-Ekbom disease (restless legs syndrome).

Rotigotine is commercially available in several formulations, one of them being that of transdermal application through patches. However, a number of side effects have been described associated to the use of rotigotine patches, for instance: somnolence, dermatitis, swelling, hallucinations, headache, diarrhea-constipation, anorexia, psychosis, compulsive disorders (gambling addiction and hyper-sexuality), convulsions, tachycardia, anxiety, among others. In addition to side effects, using these patches involves a number of drawbacks and patient discomfort as the application site for the patch must be changed every day, and repeating the same spot is contraindicated unless 14 days have elapsed between applications. Besides, a series of hygiene limitations as well as dermatological health issues concerning the application site are also associated, with the consequent stress these daily complications entail for the patient.

Gamma-amino-butyric acid (GABA) is a non proteinogenic amino acid present in microorganisms, plants and animals synthesized from glutamine and glutamate. GABA is the neurotransmitter most widely distributed throughout the human brain, and the main inhibitor in the central nervous system (brain soother). It is also responsible for the regulation of muscle tone.

GABA is found at very high concentrations in a number of areas of the brain, up to one thousand times higher than the classic monoaminergic neurotransmitters, serotonin and dopamine, in the very same regions.

This non proteinogenic amino acid is clearly found present in the cerebellum where Purkinje cells collecting the main appetites from the bulb, act releasing GABA into the thalamus and hypothalamus, into the grey basal nuclei, and into the very cerebral cortex. GABA is also found within the substantia nigra with a high concentration of dopaminergic neurons, where its high contents and that of its enzymatic systems highlight the fact that the regulation of the substantia nigra dopaminergic activity over the corpus striatum depends on GABA innervation.

Due to its tranquilizing effect, GABA is prescribed in nutritional therapies both to prevent as well as to help ease anxiety. It is also used as brain strengthener at highly stressful times and when precedents of anxiety crisis due to nervous hyper-excitability exist.

There is a wide assortment of medicines increasing the amount of GABA in the nervous system in order to induce relaxation and controlling anxiety and convulsions. *Benzodiazepins* are among such drugs, the most widely used medicines worldwide for the treatment of anxiety due to their anxiolytic, hypnotic, anti-convulsive, muscle relaxant, sleep inducer and sedative properties. Their use is so widespread that they have become the par excellence drugs of western civilization, characterized by the scourge of anxiety as a generalized pathology. Nevertheless, their massive consumption causes very sound economic and social repercussions.

Despite its generalized use, *benzodiazepines* contraindications and side effects have given rise to a broad array of medical warnings, stemming from the consequences patients allergic to such substances as well as those suffering from myasthenia gravis, shock, coma or acute alcohol poisoning face.

Furthermore, when such drugs are administered to patients with a history of drug dependency, severe respiratory failure, angle-closure glaucoma or depressive states, they are prescribed under special clinic control, with a recommended adjustment of dose when dealing with patients suffering of kidney or liver insufficiency.

In most cases adverse reactions follow after a prolonged drug treatment affecting, mostly, the central nervous system.

The consequence more widely reported in 50% of patients treated with *benzodiazepines* is sleepiness, closely followed by confusion and ataxia, especially in elderly persons and weakened patients. A high number of side effects have also been described, among which dizziness, sedation, headaches, depression, disorientation, dysphasia or dysarthria, decreased concentration, tremor, libido alterations, urinary incontinence, gagging, urinary retention, vomits, diarrhea, constipation, dry mouth, hyper-salivation and epigastric pain. Occasionally the use of *benzodiazepines* has led to hepatitis, jaundice, dermatitis, urticaria, itching, leukopenia, agranulocytosis, anaemia, thrombocytopenia, eosinophilia, behavioral disorders, amnesia, paradoxical excitement, psychosis, visual disturbances, diplopia, nystagmus, and hearing impairment, and very rarely respiratory depression, hypotension, bradycardia, tachycardia, palpitations and episodes of mania and hypomania.

It has to be added that the prolonged use of *benzodiazepines* causes habit, and a sudden interruption of a treatment at usual doses is likely to entail withdrawal syndrome (anxiety, agitation, aggressiveness, insomnia, tremor and muscle spasms). Additionally, if the treatment has involved high doses of this psychodrug, the withdrawal syndrome may be severe, with delirium and seizures.

There are certain natural substances which have been traditionally associated with the treatment of psychomental disorders for their ability to modulate the neurotransmitters associated with such disorders. Among these substances are caffeine, chocolate and glutamine, which are the subject-matter of the present invention. Nowadays it is common to find in the market food supplements that contain these substances for including them in the diet and thus treating different physiological, neurological and neurohormonal disorders. The use of such food supplements does not cause side effects. However, their efficacy in the treatment of such disorders is limited, since they are administered orally, which significantly decreases their bioavailability in the patient.

In view of the aforementioned, there is a need for products capable of efficiently modulating the concentration of neurotransmitters in the nervous system for the treatment of psychomental disorders and for which the extremely severe side effects associated with the drugs presently offered are not present.

It is relevant to highlight the fact that the use of some substances such as caffeine for transdermal administration through patches as an alternative delivery system has already been described. Prove of this are documents such as the patent GB2438926A from Paul Marshal where the use of caffeine patches to raise awareness of the user is claimed, the one from Eric T. Fossel US2015/010619A1 which discloses the use of transdermal patches and contemplates the use of substances such as caffeine in their compositions, or documents such as the United States patent US2018/243251A1 from loffe Biotechnologies Inc. where a composition of different substances including caffeine for transdermal use is described.

In this sense, the inventors of the present invention have surprisingly found that the use of transdermal patches which contain natural or active substances such as caffeine, chocolate or glutamine specifically applied on the auricle, allows to avoid the side effects of the treatment with the above mentioned drugs, while keeping a higher efficacy rate as compared to presently known routes of administration.

Therefore, the present invention refers to transdermal patches which comprise active substances capable of modulating the neurotransmitters associated with psychomental disorders to be used in a treatment known as auriculotherapy.

Auriculotherapy is based upon the existence of reflex cutaneous points with diminished electrical resistance in the auricular pavilion, which correlate to the histological microformations pertaining to a nerve, a lymphatic vessel, a small artery or venule. These microformations are called neurovascular complexes.

The auricular pavilion holds unique diagnosis and treatment properties due to its innervation and to the existence of the said neurovascular complexes. As well as in body acupuncture, in auriculotherapy a principle known as "visceral cutaneous reflex" holds true, where stimulation of the skin in a certain point in a microsystem triggers internal changes of a homeostactic nature, leading to pain relief and the healing of the organ involved. Said cutaneous stimulation launches messages from the nervous system to the spinal cord and the brain, activating bioenergetic changes leading to a biochemical release and alteration of the electric discharge in the neuronal reflexes.

The points in the auricular microsystem include, among others, master points and reference points, musculoskeletal points, those pertaining to the internal organs and neuroendocrine points.

The stimulation of reflex points in the ear or auriculotherapy has proven to be highly satisfactory in the achievement of quicker and more effective results as compared to acupuncture, both for the treatment of pain as in therapies for addictive substance abuse.

The fundamental principle of auriculotherapy lies, precisely, in the correspondence between the cartography of the auricle and pathological conditions in homologous organs of the body. Thus, auriculotherapy deals with the disease at its core, and not only with its symptomatic representation, whilst it provokes a physiological change acting jointly with the mechanisms of homeostatic self-regulation of the organism itself.

The techniques used for the stimulation of auriculotherapy points are usually acupressure, acupuncture and electroacupuncture. However, many patients feel aversion and even fear for needles or electrical stimulation. An updated alternative involves laser beams to stimulate those points, or placing semi-permanent seeds on the auricle. Nevertheless, such techniques still represent a certain degree of discomfort for the patient and can eventually entail physiological and psychological limitations.

Conversely, the use of transdermal patches applied on the auricle does not have discomfort or limitations for the patient.

Therefore, and in a first place, the present invention provides a transdermal patch for use in auriculotherapy comprising at least one active substance for the modulation of neurotransmitters, selected said active substance among caffeine, chocolate and glutamine.

Caffeine (1, 3,7-Trimethylxanthine), which is the main ingredient of coffee, is a psychoactive substance whose stimulating properties stem from the antagonistic role of adenosine A(1) and adenosine A(2) receptors. Caffeine's ability to reduce the transmission of adenosine in the brain has turned it into the most widely consumed psychoactive substance worldwide, as it induces the release of dopamine into the cortex of the nucleus accumbens. Coffee is also regarded as a substance protective of the brain, as well as playing an important role in the caring of intestinal flora (microbiota).

Caffeine weighs on neuronal physiology as it improves attention levels, alertness, reaction or physical performance at muscular strength and resilience levels. On a continued consumption basis, it also serves as effective strategy to keep up physical and cognitive capacities. Recent studies have lately shown that caffeine can dynamically alter the neuronal elements influencing the behavior of neurotransmitters, as it clearly stimulates dopaminergic cells in the brain.

Additionally, both chocolate and its base, cocoa are food containing a high amount of minerals such as magnesium, potassium, chromium, zinc, phosphorus or iron, vitamins A, B, C or E, other substances such as polyphenols and flavonoids, and amino acids, with the essential amino acid tryptophan standing out, as well as other alkaloids such as theobromine, anandamide and phenethyilamine which contribute to the feeling of peacefulness and happiness. Chocolate or cocoa consumption is recommended in cases of mineral deficiency in the diet due to its high concentration of minerals, but also for the regulation of mood, food ingestion and compulsive disorders, as it contributes to balance low levels of neurotransmitters, stimulating those of serotonin and dopamine at the nucleus accumbens.

Both serotonin as well as its precursor tryptophan are present in commercial formulations of chocolate with high percentages of cocoa, where the greatest percentages of serotonin are found in chocolates with a content of cocoa equal to or greater than 85%.

These high levels of serotonin, together with the antioxidant (flavonoids) and anti-inflammatory agents contained in chocolate, are responsible for its proven benefits to cardiovascular health as well as for the prevention of insulin resistance. For these reasons both cocoa and chocolate are among the most attractive substances for the natural treatment of health disorders.

Many studies have demonstrated that the consumption of polyphenol rich foods help decrease oxidative stress, which in turn reduces substantially the risk of neurological conditions. The major sources of polyphenols include fruits, vegetables and, as noted above, cocoa and coffee. In addition, the intake of coffee and cocoa derived flavonoids (a kind of antioxidant polyphenol) reduces insulin resistance and blood pressure, hence fostering the stimulation of neurotransmitters, which will be less affected by free radicals therefore improving cognitive function.

Glutamine, on the other hand, is a non-essential amino acid closely related to glutamic acid and gamma-amino-butyric acid (GABA), all of them performing their neurotransmitter metabolic functions as a team. Glutamine behaves, mainly, as a natural inhibitor or relaxant, and it is known as the great "natural balancer" of excitement and lethargy. Glutamine can be ingested via diet, as it is naturally present in eggs, dry fruits, fish, meat, dairy and its byproducts.

Glutamine is a source of energy for the mind, helping fight mental fatigue and numbness. It also aids people suffering from hypoglycemia (low sugar levels in the blood), one of the most important causes of memory decay, hence improving learning, retaining and remembering capacities.

Another one in the many benefits of glutamine arises from its property of helping control addiction to alcoholic beverages, carbonated soft drinks and sweets, as it reduces resistance to insulin which blocks its stimulation, thus naturally enhancing the levels of the neurotransmitters dopamine, serotonin and GABA. Besides, glutamine reduces fat build-up and increases the production of growth hormone, in turn resulting in a rise in muscle tone and thus becoming highly recommendable to delay ageing.

Glutamine inhibits the accumulation of ammonia in the brain and reduces neuromuscular damage resulting from such accumulation. As a precursor for glutathione, it also multiplies the body defenses against oxidative damage via glutathione peroxidase.

As previously stated, it has been surprisingly discovered that the specific application on the auricle of the transdermal patches of the present invention, comprising at least one active substance for modulating neurotransmitters selected from among caffeine, chocolate and glutamine, allows the optimal stimulation of the relevant reflex point in order to trigger a series of stimuli in the neurological system and in the glands pertaining to the hypothalamic-pituitary axis, thus generating microhormonal substances capable of regulating the endocrine system and, consequently, the levels of dopamine, serotonin and GABA in the individual thereby treated.

The transdermal patches for auriculotherapy of the present invention are devices of a therapeutic-nutritional-orthomolecular kind that enable, in an effective manner, the modulation of the concentration of the key neurotransmitters involved in pathologies and physiological, neurological and neurohormonal disorders, taking advantage of the electro sensitive points at the auricle to receive the stimulation of the active substance in a safe and harmless manner and with no side effects to the patient, and with no inconvenience for the latter arising from the therapeutic action, as opposed to other approaches described in the prior art.

Furthermore, the application of the transdermal patches for auriculotherapy of the present invention entails other advantages for the patient: it is simple and practical, cost-effective, the subject is not compelled to get undressed, which averts any emotional stress during the therapeutic session; it is non-invasive and, at the auricle, the risks of damaging any internal system are minimal, if not non-existent.

The inventors of the present invention have discovered that the use of the transdermal patches of the present invention, belonging to the group so-called transdermal therapeutic systems (TTS), characterized by their ability to provide a sustained release of the active substance, enables an optimal release of the substances of interest (such as caffeine, chocolate or glutamine, or derivative thereof) in the patient since its specific application on the auricle enables the molecular diffusion of said active substances from the stratum corneum through the epidermis, in favor of a concentration gradient, entering the blood stream where it reaches a constant plasma concentration thus rendering a systemic effect.

In one preferred embodiment, the transdermal patch for auriculotherapy of the present invention can comprise an active substance for the modulation of, at least, one neurotransmitter. In a preferable embodiment, the transdermal patch for auriculotherapy of the present invention can comprise several active substances for the modulation of one or several neurotransmitters. It is thus contemplated the combination of several active substances to act jointly upon a single neurotransmitter and/or several active substances to act jointly upon different neurotransmitters. In a preferable embodiment, the present invention is a transdermal patch for auriculotherapy which contains at least one active substance selected from caffeine, chocolate and glutamine for the modulation of a distinct neurotransmitter.

A person skilled in the art knows which sources may contain the active substances above described, namely, coffee, tea, guarana, mate tea, chocolate, cocoa, milk, eggs, nuts, meat and fish, among others. Thus, the transdermal patch for use in auriculotherapy of the present invention may comprise any of said substances that are natural sources of the active substances for the modulation of neurotransmitters.

In a preferred embodiment, the transdermal patch for use in auriculotherapy of the present invention comprises caffeine or a derivative thereof, for the modulation of neurotransmitters.

In another preferred embodiment, the transdermal patch for use in auriculotherapy of the present invention comprises chocolate or a derivative thereof, for the modulation of neurotransmitters. In a more preferred embodiment, the transdermal patch for use in auriculotherapy of the present invention comprises cocoa of any origin in any of the forms known to the person skilled in the art, or cocoa derivatives such as cocoa paste, cocoa butter or cocoa powder, thus comprising at least one active substance of those found naturally in cocoa such as tryptophan, anandamide, phenylethylamine, polyphenols or flavonoids, for the modulation of neurotransmitters. In an even more preferred embodiment, the transdermal patch for use in auriculotherapy of the present invention comprises at least one active substance that is naturally present in cocoa such as tryptophan, anandamide, phenylethylamine, polyphenols or the flavonoids, for the modulation of neurotransmitters.

In another more preferred embodiment, the transdermal patch for use in auriculotherapy of the present invention comprises chocolate in any of the forms known to the person skilled in the art, such as dark chocolate, milk chocolate or white chocolate. In another more preferred embodiment, the chocolate contains at least 50% cocoa, at least 60% cocoa, at least 70% cocoa, at least 75% cocoa, at least 80% cocoa, at least 85% cocoa, at least 90% cocoa, at least 95% cocoa, at least 97% cocoa, at least 98% cocoa or at least 99% cocoa. In another more preferred embodiment, the chocolate of the transdermal patch of the present invention contains 100% cocoa.

In another preferred embodiment, the transdermal patch for use in auriculotherapy of the present invention comprises glutamine or a derivative thereof, for the modulation of neurotransmitters.

In another preferred embodiment, the transdermal patch for use in auriculotherapy of the present invention comprises a combination of at least two active substances or ingredients selected from the group consisting of chocolate, cocoa, or an active substance that is naturally present in chocolate or cocoa, such as tryptophan, theobromine, anandamide, phenylethylamine, polyphenols or flavonoids, caffeine or a derivative thereof, and glutamine or a derivative thereof, for the modulation of neurotransmitters.

In a preferred embodiment, the transdermal patch for auriculotherapy can also comprise at least one further ingredient or cofactor. Such further ingredient or cofactor can increase the efficiency of the active substance and is chosen from the list comprising:
L-tyrosine, phenylalanine, 5-htp decarboxylase, GABA transaminase, theanine, 5-hydroxy-tryptophane, tryptophan, phenylethylamine, theobromine, anandamide, gamma hydroxybutyrate acid, gamma hydroxybutyric acid, taurine, homotaurine, melatonin, carnitine, acetyl-carnitine, S-Adenosylmethionine, N-acetyl L-cysteine, N-acetyl-transferase, phosphatidylserine, inositol, phosphatidylinositol, catalase, dopamine beta-hydroxylase, L-dopa descarboxylase, NADH, magnesium, zinc, manganese, chromium, copper, selenium, iron, calcium, lithium, calcium citrate, vitamin C, vitamins B, coenzyme Q10, omega-3, ashwagandha, Melissa oficinalis, Siberian ginseng, St. John's wort, valerian, Ginko biloba, Mucuna pruriens, turmeric, Rhodiola rosea and Bacopa monniera extracts, or a combination thereof.

In another preferred embodiment, the further ingredient or cofactor of the transdermal patch for auriculotherapy according to the present invention may be an active substance that is naturally present in cocoa, such as tryptophan, theobromine, anandamide, phenylethylamine, polyphenols or flavonoids, for the modulation of neurotransmitters.

The active substance comprised in the transdermal patch for auriculotherapy of the present invention will be chosen according to the pathology or disorder the patient to be treated with is suffering from. A person skilled in the art knows what active substances are apt to modulate the neurotransmitters involved in each kind of pathology or disorder and, therefore, is able to determine the active substance adequate for every case.

In the case of multi-factorial disorders or pathologies where modulating several neurotransmitters is deemed necessary and combining diverse active substances in a unique transdermal patch is not possible, the use of various transdermal patches for auriculotherapy according to the present invention is contemplated, each of them being capable of comprising one or several active substances, and one or several of the further ingredients or cofactors mentioned above.

The active substances comprised in the transdermal patch for auriculotherapy of the present invention can be found in said patch at a concentration between 1% by weight up to 90% by weight relative to the total of components. More preferably, the active substance or substances included in the transdermal patch of the present invention are at a concentration between 1% by weight and 50% by weight with respect to the total components. In a preferred embodiment, the active substance in the transdermal patch for auriculotherapy according to the present invention will be present in an amount ranging between 0.01 and 1000 mg, more preferably between 0.1 and 750 mg, and even more preferably between 0.15 and 500 mg. Further still, more preferably between 0.2 and 250 mg and still more preferably between 0.5 and 100 mg and, the most preferable, between 1 and 50 mg.

Preferably, the at least one further ingredient or cofactor comprised in the transdermal patch for auriculotherapy of the present invention can be found in said patch at a concentration between 1% by weight up to 90% by weight relative to the total of components. More preferably, at least one further ingredient or cofactor comprised in the transdermal patch of the present invention is at a concentration between 1% by weight and 50% by weight with respect to the total components. In a preferred embodiment, the further ingredient or cofactor in the transdermal patch for auriculotherapy according to the present invention will be present in an amount ranging between 0.01 and 1000 mg, more preferably between 0.1 and 750 mg, and even more preferably between 0.15 and 500 mg. Further still, more preferably between 0.2 and 250 mg and still more preferably between 0.5 and 100 mg and, the most preferable, between 1 and 50 mg.

Either way, the concentration of the at least one active substance and the further ingredient/cofactor or further ingredients/cofactors in the transdermal patch for auriculotherapy of the present invention will vary according to the desired dose, the permeability of the materials employed in the manufacture of the patch or the duration of the treatment. A person skilled in the art will be able to establish such concentrations according to the disorder or pathology of the patient to be treated.

Besides the active substances above detailed, the transdermal patch for auriculotherapy of the present invention can comprise one or several excipients. Among the excipients that the transdermal patch for auriculotherapy of the present invention can comprise are bonding agents, penetration promoting agents, thinners, disintegrants, lubricants, coaters, and flavoring and/or coloring agents, among others.

The transdermal patch according to the present invention can comprise several structural elements. A person skilled in the art is capable of determining the structural elements of the transdermal patch according to the present invention based upon the active substance of the patch and the treatment or pathology of the patient to be treated therewith. In a preferred embodiment, the transdermal patch for auriculotherapy comprises the following structural elements:
- a protective film or cover.
- a deposit containing at least one active substance,
- a self-adhesive surface for fixing the patch to the skin, and/or
- a release controlling system,

In a preferred embodiment, the transdermal patch of the present invention has a self-adhesive surface for fixing the patch to the skin, which acts as deposit of at the least one active substance as well as of the rest of ingredients/cofactors and/or excipients. In another preferred embodiment, the release controlling system is also contained within the self-adhesive surface which acts as deposit of the active substances.

The transdermal patch for auriculotherapy of the present invention can be manufactured from different materials known in the state of the art. Examples of such materials are cellulose acetate, ethyl cellulose, polyethylene terephthalate, plastified copolymers of vinyl acetate and vinyl chloride, nylon, ethylene and vinyl acetate copolymers, plasticised polyvinyl chloride, polyurethane, polyvinylidene chloride, polypropylene, polyethylene, polyamide or aluminum, among others. The choice of the material for the manufacture of the different structural elements or layers of the transdermal patch of the present invention is not relevant for the technical effect thereof and as indicated above, the person skilled in the art knows the most suitable available materials for each type of active substance.

However, in a preferred embodiment, the transdermal patch for auriculotherapy according to the present invention can be manufactured with a self-adhesive surface based on polyacrylate-type polymers (acrylic acid polymers) or polyisobutylene or silicone. The patch can also comprise an upper layer bonded to the self-adhesive surface, which acts as a protective film or cover. This layer can be impermeable to the active substance and have an occlusive nature. Any material out of those used in conventional preparations as known to a person skilled in the art can be utilized at will, such as the materials described above, for the manufacture of the layer which acts as a protective film or cover.

In another preferred embodiment of the present invention, the transdermal patches for auriculotherapy have the necessary elements to be applied to the patient by passive iontophoresis technique.

lontophoresis is a technique of transdermal administration of active substances of interest by using low intensity electric current gradient. On the other hand, passive iontophoresis is a modification of said technique in which the application of an external electric current is not carried out. Thus, the transdermal patches for application by passive iontophoresis of the present invention include voltaic cells which generate an electromagnetic field that allows the ions of the active substances of interest to penetrate more efficiently into the skin. In addition, the electro-ionic action creates a "cellular massage" that favors the exchange of oxygen between the cells, revitalizing the connective tissue.

Thus, the transdermal patches for auriculotherapy by passive iontophoresis of the present invention comprise at least:
- an outer layer of tissue containing alternating copper and zinc voltaic cells.
- a layer or adhesive surface containing the active substances.

Said voltaic cells generate ions of the active substances present in the transdermal patches of the present invention, said ions being induced by the low intensity current that is self-generated at the contact of the patch and sweat. Thanks to the occlusive effect of the patch, the evaporation of sweat fluids takes place, and moisten the adhesive and the tissue, creating microcurrents and a magnetic microfield that contributes to generating the ionic substances that, due to their electric charge, tend to move towards the pole of opposite charge, where they are absorbed more easily through the skin. In this way, passive iontophoresis improves the absorption of substances with respect to conventional topical use.

Therefore, the transdermal patches for auriculotherapy according to the present invention can be manufactured specifically for application by the technique of passive iontophoresis or manufactured for application by simple molecular diffusion in favor of a concentration gradient.

According to the invention, the total area of the transdermal patch for auriculotherapy according to the present invention may vary between 10 and 200 mm². More preferably, the total area is between 25 and 175 mm², even more preferably between 50 and 150 mm², even more preferably between 50 and 100 mm².

Traditional transdermal patches, known to those skilled in the art for application on parts of the body other than the auricle, are larger than the transdermal patches for auriculotherapy of the present invention, which have a total area between 10 and 200 mm². This reduced size makes them more adapted for their specific application on the auricle, following the technique of auriculotherapy, compared to traditional patches, which due to their larger size, cannot be effectively applied on said surface.

The shape of the transdermal patch for auriculotherapy according to the present invention can be square, round, oval, triangular, elliptical or any whatsoever shape known to a person skilled in the art.

The transdermal patches for auriculotherapy according to the present invention can be applied to different areas of the auricle, depending on the reflex point/points to be stimulated by the relevant active substance. The precise location for the application of said patches is determined by the specific neurotransmitter to be regulated and a person skilled in the art is capable of doing so. For example, transdermal patches for auriculotherapy according to the present invention can be applied on the lobe, the swallow, in antitrago, the shell, the antehelix, the helix, the navicular fossa, the scaphoid fossa, the triangular fossa or the upper branch of the antehelix, among others.

Finally, the present invention relates to a method for treating physiological, neurological and neurohormonal disorders by means of auriculotherapy using a transdermal patch, as stated above.

Among said physiological, neurological and neurohormonal disorders are depression, eating disorders such as bulimia and anorexia, disorders involving addiction to toxic substances, mental disorders such as schizophrenia, appetite regulation and overweight control, increase of libido and regulation of prolactin levels, regulation of motor activity and cognitive functions, of learning and memory, attention deficits, treatment of pain as anaesthetic, to control nausea and vomiting and even to improve immunity, for insomnia, to control panic attacks, stress, aggressiveness, tobacco use, neurological diseases such as Parkinson's, headaches and migraines, for regulating circadian rhythm, gastrointestinal movement and neuroendocrine functions, for the treatment of irritable bowel, pain in chronic cases such as fibromyalgia, for helping relaxation, or for the treatment of post-traumatic stress, and as anxyolitic, anti-convulsive, muscle relaxant, sedative and/or anti-hypertensive, among others.

The total duration of treatment may vary depending on the disorder or pathology being treated, as well as the active substance or substances involved. In a preferred embodiment of the present invention, the total duration of treatment by means of transdermal patches for auriculotherapy may last between 2 weeks and 18 months. More preferably the treatment may last between 4 weeks and 12 months. A person skilled in the art is capable of determining the duration of the treatment according to the pathology or disorder being treated as well as the active substances chosen for such treatment.

On the other hand, the application time of each patch of the present invention can also vary depending on the disorder or pathology to be treated, as well as the active substance or substances included therein. In one embodiment of the present invention, the application time of each transdermal patch for auriculotherapy according to the present invention varies between 12 hours and 12 weeks. More preferably, said application time varies between 24 hours and 10 weeks, more preferably between 48 hours and 5 weeks, more preferably between 3 days and 21 days.

### Examples

Example 1: Preparation of the transdermal patches for auriculotherapy containing an active substance according to the present invention.

Square-shaped transdermal patches of a size of 100 mm² comprising three layers were prepared: a layer containing the release controlling system, a silicone adhesive layer containing the deposit for the active substance, and a protective occlusive layer.

Four different active substances were used for the manufacture of four different types of transdermal patches: caffeine, chocolate (dark powder type), tryptophan and glutamine.
a) A solution containing a final concentration of 600 mg/ml of caffeine was prepared in the appropriate solvent. 1 ml of said solution was added to the deposit located in the adhesive layer and was allowed to dry. Subsequently, said adhesive layer was attached on one side to the release controlling system layer and sealed on the other side with the protective occlusive layer.
b) A solution containing a final concentration of 750 mg/ml of dark chocolate powder was prepared in the appropriate solvent. 1 ml of said solution was added to the deposit located in the adhesive layer and was allowed to dry. Subsequently, said adhesive layer was attached on one side to the release controlling system layer and sealed on the other side with the protective occlusive layer.
c) A solution containing a final concentration of 100 mg/ml tryptophan was prepared in the appropriate solvent. 1 ml of said solution was added to the deposit located in the adhesive layer and was allowed to dry. Subsequently, said adhesive layer was attached on one side to the release controlling system layer and sealed on the other side with the protective occlusive layer.
d) A solution containing a final concentration of 500 mg/ml of glutamine was prepared in the appropriate solvent. 1 ml of said solution was added to the deposit located in the adhesive layer and was allowed to dry. Subsequently, said adhesive layer was attached on one side to the release controlling system layer and sealed on the other side with the protective occlusive layer.

The patches were stored in containers of 30 patches each and their stability at room temperature was checked from 6 to 36 months.

Example 2: Preparation of the transdermal patches for auriculotherapy containing two active substances according to the present invention.

Square-shaped transdermal patches of a size of 50 mm² comprising three layers were prepared: a layer containing the release controlling system, a polyacrylate adhesive layer containing the deposit for the active substances, and a protective occlusive layer.

Two groups of different active substances were used for the manufacture of transdermal patches of combined action for auriculotherapy: a) chocolate (of the dark powder type) and tryptophan, and b) defatted cocoa powder, tryptophan and magnesium.
a) A solution containing a final concentration of 750 mg/ml of dark chocolate powder and 100 mg/ml of tryptophan was prepared in the appropriate solvent. 1 ml of said solution was added to the deposit located in the adhesive layer and was allowed to dry. Subsequently, said adhesive layer was attached on one side to the release controlling system layer and sealed on the other side with the protective occlusive layer.
b) A solution containing a final concentration of 450 mg/ml of defatted cocoa powder, 150 mg/ml of tryptophan and 45 mg/ml of magnesium solvent was prepared in the appropriate. 1 ml of said solution was added to the deposit located in the adhesive layer and was allowed to dry. Subsequently, said adhesive layer was attached on one side to the release controlling system layer and sealed on the other side with the protective occlusive layer.

The patches were stored in containers of 30 patches each and their stability at room temperature was checked from 6 to 36 months.

Example 3: Preparation of the transdermal patches for auriculotherapy containing an active substance and a cofactor according to the present invention.

Square-shaped transdermal patches of a size of 75 mm² comprising three layers were prepared: a polyacrylate adhesive layer containing the deposit for the active substances and the release controlling system, and a protective occlusive layer.

The following groups of active substances and different cofactors were used for the manufacture of transdermal patches for auriculotherapy: a) caffeine and vitamin C, b) dark chocolate powder and magnesium, c) tryptophan and magnesium, and d) glutamine and inositol.
a) A solution containing a final concentration of 500 mg/ml of caffeine and 250 mg/ml of vitamin C was prepared in the appropriate solvent. 1 ml of said solution was added to the deposit located in the adhesive layer and was allowed to dry. Subsequently, said adhesive layer was sealed on the other side with the protective occlusive layer.
b) A solution containing a final concentration of 700 mg/ml of dark chocolate powder and 200 mg/ml of magnesium was prepared in the appropriate solvent. 1 ml of said solution was added to the deposit located in the adhesive layer and was allowed to dry. Subsequently, said adhesive layer was sealed on the other side with the protective occlusive layer.
c) A solution containing a final concentration of 250 mg/ml tryptophan and 100 mg/ml magnesium was prepared in the appropriate solvent. 1 ml of said solution was added to the deposit located in the adhesive layer and was allowed to dry. Subsequently, said adhesive layer was sealed on the other side with the protective occlusive layer.
d) A solution containing a final concentration of 500 mg/ml of glutamine and 100 mg/ml of inositol was prepared in the appropriate solvent. 1 ml of said solution was added to the deposit located in the adhesive layer and was allowed to dry. Subsequently, said adhesive layer was sealed on the other side with the protective occlusive layer.

The patches were stored in containers of 30 patches each and their stability at room temperature was checked from 6 to 36 months.

Example 4: Preparation of the transdermal patches for auriculotherapy containing an active substance or a combination of an active substance and a cofactor, by passive iontophoresis according to the present invention.

Square-shaped transdermal patches of a size of 100 mm² comprising two layers were prepared: a polyacrylate adhesive layer containing the deposit for the active substance alone or in combination with a cofactor, and a protective layer containing the alternating voltaic cells of Copper and Zinc.

As active substances, caffeine, dark chocolate powder, tryptophan and glutamine and as cofactors respectively vitamin C, magnesium, magnesium again, and inositol were used for the manufacture of transdermal patches for auriculotherapy by passive iontophoresis.
a) A solution containing a final concentration of 500 mg/ml of caffeine was prepared in the appropriate solvent. 1 ml of said solution was added to the deposit located in the adhesive layer and was allowed to dry. Subsequently, said layer was sealed with the protective layer.
b) A solution containing a final concentration of 750 mg/ml of dark chocolate powder was prepared in the appropriate solvent. 1 ml of said solution was added to the deposit located in the adhesive layer and was allowed to dry. Subsequently, said layer was sealed with the protective layer.
c) A solution containing a final concentration of 250 mg/ml tryptophan was prepared in the appropriate solvent. 1 ml of said solution was added to the deposit located in the adhesive layer and was allowed to dry. Subsequently, said layer was sealed with the protective layer.
d) A solution containing a final concentration of 450 mg/ml of glutamine was prepared in the appropriate solvent. 1 ml of said solution was added to the deposit located in the adhesive layer and was allowed to dry. Subsequently, said layer was sealed with the protective layer.
e) A solution containing a final concentration of 500 mg/ml of caffeine and 250 mg/ml of vitamin C was prepared in the appropriate solvent. 1 ml of said solution was added to the deposit located in the adhesive layer and was allowed to dry. Subsequently, said layer was sealed with the protective layer.
f) A solution containing a final concentration of 700 mg/ml of dark chocolate powder and 500 mg/ml of magnesium was prepared in the appropriate solvent. 1 ml of said solution was added to the deposit located in the adhesive layer and was allowed to dry. Subsequently, said layer was sealed with the protective layer.
g) A solution containing a final concentration of 250 mg/ml tryptophan and 100 mg/ml magnesium was prepared in the appropriate solvent. 1 ml of said solution was added to the deposit located in the adhesive layer and was allowed to dry. Subsequently, said layer was sealed with the protective layer.
h) A solution containing a final concentration of 500 mg/ml of glutamine and 100 mg/ml of inositol was prepared in the appropriate solvent. 1 ml of said solution was added to the deposit located in the adhesive layer and was allowed to dry. Subsequently, said layer was sealed with the protective layer.

The patches were stored in containers of 30 patches each and their stability at room temperature was checked from 6 to 24 months.

Example 5: Preparation of the transdermal patches for application on the arm containing an active substance according to the prior art.

Transdermal patches for application in the patient's arm according to the prior art were prepared. Said patches, rectangular and with a size of 10 cm², comprised three layers: a polyacrylate adhesive layer containing the deposit for the active substances and the release controlling system, and a protective occlusive layer.

The same active substances used for the transdermal patches for auriculotherapy of the present invention were used for the manufacture of three transdermal patches according to the prior art and: caffeine, dark chocolate powder, tryptophan and glutamine.
a) A solution containing a final concentration of 300 mg/ml of caffeine was prepared in the appropriate solvent. 2 ml of said solution was added to the deposit located in the adhesive layer and was allowed to dry. Subsequently, said adhesive layer was attached on one side to the release controlling system layer and sealed on the other side with the protective occlusive layer.
b) A solution containing a final concentration of 375 mg/ml of dark chocolate powder was prepared in the appropriate solvent. 2 ml of said solution was added to the deposit located in the adhesive layer and was allowed to dry. Subsequently, said adhesive layer was attached on one side to the release controlling system layer and sealed on the other side with the protective occlusive layer.
c) A solution containing a final concentration of 50 mg/ml tryptophan was prepared in the appropriate solvent. 2 ml of said solution was added to the deposit located in the adhesive layer and was allowed to dry. Subsequently, said adhesive layer was attached on one side to the release controlling system layer and sealed on the other side with the protective occlusive layer.
d) A solution containing a final concentration of 250 mg/ml glutamine was prepared in the appropriate solvent. 2 ml of said solution was added to the deposit located in the adhesive layer and was allowed to dry. Subsequently, said adhesive layer was attached on one side to the release controlling system layer and sealed on the other side with the protective occlusive layer.

Example 6: Comparative study of a treatment with the transdermal patches by auriculotherapy according to the present invention compared to a treatment with transdermal patches according to the prior art, for the treatment of carbohydrate abuse (sugars).

A total of 18 patients aged between 45 and 55, diagnosed with obesity due to abuse of carbohydrates (sugars) through anamnesis and initial urinalysis, which showed low levels of serotonin (between 90 and 120 µg/24h), slightly low levels of dopamine (between 100 and 118 µg/24h), and slightly high levels of GABA (between 4.8 and 5.5 µg/24h), were treated with the transdermal chocolate patches as described below.

The patients were divided into the following groups:
- Group I: 3 patients were treated with transdermal patches containing chocolate, such as those described in example 1b), using the auriculotherapy technique.
- Group II: 3 patients were treated with transdermal patches of chocolate and magnesium, such as those described in example 3b), using the auriculotherapy technique.
- Group III: 3 patients were treated with transdermal patches of chocolate by iontophoresis, such as those described in example 4b), using the auriculotherapy technique.
- Group IV: 3 patients were treated with transdermal patches of chocolate and magnesium by iontophoresis, such as those described in example 4f), using the auriculotherapy technique.
- Group V: 3 patients were treated with transdermal patches of chocolate applied on the arm, such as those described in example 5b).
- Group VI: 3 patients were treated with transdermal patches that did not contain any active substances, but were manufactured in a manner similar to the patches of Example 1.

The 18 patients were treated with the transdermal patch of chocolate indicated for their group for a period of seven weeks. Each patient had a patch placed on the auricle or arm, as appropriate, which was maintained during the seven weeks of the treatment. The aim of the therapeutic approach was to increase serotonin levels and consequently, increase the slightly reduced levels of dopamine and decrease the slightly high levels of GABA.

After seven weeks of treatment, the availability of serotonin and dopamine within the presynaptic vesicles was checked by means of a new urine test. The average results obtained for each of the groups are the following:

| | Group I | Group II | Group III | Group IV | Group V | Group VI |
|---|---|---|---|---|---|---|
| Serotonin (µg/24h) | 165-184 | 170-193 | 175-201 | 192-225 | 95-103 | 89-119 |
| Dopamine (µg/24h) | 135-165 | 145-163 | 150-172 | 150-175 | 105-130 | 100-120 |
| GABA (µg/24h) | 4-4.6 | 3.9-4.2 | 2.1-3.8 | 3-4.1 | 4.9-5.5 | 5-5.6 |

The results obtained in urine tests revealed the efficacy of the transdermal patches for auriculotherapy of the present invention. Thus, the groups treated with the auriculotherapy patches according to the present invention with at least chocolate as active substance (groups I to IV) shown an increase in the levels of serotonin and dopamine in urine, as well as a decrease below 4.5 µg/24h of GABA levels. In addition, the levels of serotonin and dopamine were slightly higher in groups III and IV, which were treated with transdermal patches for auriculotherapy using iontophoresis, as compared with the groups treated with transdermal patches for auriculotherapy without iontophoresis.

In addition, the recovery of all patients in groups I, II, III and IV was noted in the clinical consultation, and their signs of anxiety about the abusive consumption of carbohydrates (sugars) were no longer present. However, only one patient in group V perceived improvement in symptoms and none of the patients in control group VI experienced changes during treatment.

No side effects associated with the transdermal patches for auriculotherapy according to the present invention were shown in any of the groups, and only one of the patients of group V indicated discomfort by wearing a large transdermal patch visible on the arm.

Example 7: Comparative study of a treatment with the transdermal patches by auriculotherapy according to the present invention compared to a treatment with transdermal patches according to the prior art, for the treatment of mild depression.

A total of 18 patients aged between 25 and 35, diagnosed with mild depression through anamnesis and initial urinalysis, which showed low levels of serotonin (between 90 and 115 µg/24h), low levels of dopamine (between 85 and 115 µg/24h), and high levels of GABA (between 7.3 and 14 µg/24h), were treated with the transdermal patches as described below.

In this case, a combined treatment of patches containing chocolate for seven weeks with patches containing caffeine for the subsequent four weeks was chosen.

The patients were divided into the following groups:
- Group I: 3 patients were first treated with transdermal patches of chocolate, such as those described in example 1b), and subsequently with transdermal patches of caffeine, such as those described in example 1a), both using the auriculotherapy technique.
- Group II: 3 patients were treated with transdermal patches of chocolate and magnesium, such as those described in example 3b), and subsequently with transdermal patches of caffeine and vitamin C, such as those described in example 3a), both by the auriculotherapy technique.
- Group III: 3 patients were first treated with transdermal patches of chocolate by iontophoresis, such as those described in example 4b), and subsequently with transdermal patches of caffeine by iontophoresis, such as those described in example 4a), both using the auriculotherapy technique.
- Group IV: 3 patients were first treated with transdermal patches of chocolate and magnesium by iontophoresis, such as those described in example 4f), and subsequently with transdermal patches of caffeine and vitamin C by iontophoresis, such as described in example 4e), both using the auriculotherapy technique.
- Group V: 3 patients were first treated with transdermal patches of chocolate, applied on the arm, such as those described in example 5b) and subsequently with transdermal patches of caffeine applied on the arm, such as those described in example 5a).
- Group VI: 3 patients were first treated with transdermal patches that did not contain any active substances but were manufactured in a manner similar to the patches of Example 1.

In the first part of the treatment, the 18 patients were treated with the transdermal patch of chocolate indicated for their group for a period of seven weeks. Each patient had a patch placed on the auricle or arm, as appropriate, which was maintained during the seven weeks of the first part of the treatment. The aim of the therapeutic approach was to increase serotonin levels to increase dopamine levels accordingly.

In the second part of the treatment, the 18 patients were treated with the transdermal patch of caffeine indicated for their group for a period of four weeks, in order to regulate the dopamine levels.

After four weeks of this second part of the treatment, the availability of serotonin and dopamine within the presynaptic vesicles was checked by means of a new urine test. The average results obtained for each of the groups were the following:

| | Group I | Group II | Group III | Group IV | Group V | Group VI |
|---|---|---|---|---|---|---|
| Serotonin (µg/24h) | 175-199 | 182-201 | 188-219 | 197-225 | 100-120 | 92-115 |
| Dopamine (µg/24h) | 125-155 | 130-168 | 150-172 | 162-175 | 89-122 | 85-113 |
| GABA (µg/24h) | 3.5 | 3.7-4.7 | 3-3.9 | 3-4 | 6.5-8 | 6-12 |

The results obtained in urine tests revealed the efficacy of the transdermal patches for auriculotherapy of the present invention. Thus, the groups that were treated first with the patches of chocolate and subsequently of caffeine (groups I to IV) for auriculotherapy according to the present invention, all presented an increase in the levels of serotonin and dopamine in urine, as well as a decrease below 4.5-5 µg / 24h of GABA levels. In addition, the levels of serotonin and dopamine were slightly higher in groups III and IV, which were treated with transdermal patches for auriculotherapy using iontophoresis, as compared with the groups treated with transdermal patches for auriculotherapy without iontophoresis.

In addition, the recovery of all patients in groups I, II, III and IV was noted in the clinical consultation, and their signs and symptoms of depression were no longer present. However, none of the patients in group V or control group VI experienced changes in their mood during treatment.

None of the groups showed side effects associated with the transdermal patches for auriculotherapy according to the present invention.

Example 8: Comparative study of a treatment with the transdermal patches by auriculotherapy according to the present invention compared to a treatment with transdermal patches according to the prior art, for the treatment of anxiety and insomnia.

A total of 24 patients aged between 35 and 50, diagnosed with anxiety and insomnia through anamnesis and initial urinalysis, which showed imbalances in neurotransmitter levels, with low levels of serotonin (between 90 and 115 µg/24h), high levels of dopamine (between 280 and 500 µg/24h) and high levels of GABA (between 16.7 and 30 µg/24h), were treated with the transdermal patches as described below.

In this case, a treatment with patches containing chocolate for eight weeks and with patches containing glutamine for three more weeks was chosen.

The patients were divided into the following groups:
- Group I: 4 patients were first treated with transdermal patches containig chocolate, such as those described in example 1b), and subsequently with transdermal glutamine patches, such as those described in example 1d ), both using the auriculotherapy technique.
- Group II: 4 patients were first treated with transdermal patches of chocolate and magnesium, such as those described in example 3b), and subsequently with transdermal patches of glutamine and inositol, such as those described in the 3d example), both using the auriculotherapy technique.
- Group III: 4 patients were first treated with transdermal patches of chocolate by iontophoresis, such as those described in example 4b), and subsequently with transdermal patches of glutamine by iontophoresis, such as those described in example 4d), both using the auriculotherapy technique.
- Group IV: 4 patients were first treated with transdermal patches of chocolate and magnesium by iontophoresis, such as those described in example 4f), and subsequently with transdermal patches of glutamine and inositol by iontophoresis, such as those described in example 4h), both using the auriculotherapy technique.
- Group V: 4 patients were first subjected to a treatment with transdermal patches of chocolate, applied on the arm, such as those described in example 5b) and subsequently with transdermal patches of glutamine applied on the arm, such as those described in example 5d).
- Group VI: 4 patients were treated with transdermal patches that did not contain any active substances but were manufactured in a manner similar to the patches of example 1.

The 24 patients treated with the transdermal patch of chocolate indicated for their group for a period of eight weeks. Each patient had a patch placed on the auricle or arm, as appropriate, which was maintained during the eight weeks of the treatment. The aim of the therapeutic approach was to reduce the level of dopamine by increasing serotonin.

In the second part of the treatment, the 24 patients were treated with the transdermal glutamine patch indicated for their group for a period of three weeks to regulate the GABA levels.

After the last three weeks, the availability of serotonin and dopamine within the presynaptic vesicles was checked by means of a new urine test. The average results obtained for each of the groups are the following:

| | Group I | Group II | Group III | Group IV | Group V | Group VI |
|---|---|---|---|---|---|---|
| Serotonin (µg/24h) | 170-185 | 178-195 | 193-222 | 200-225 | 99-127 | 90-115 |
| Dopamine (µg/24h) | 162-175 | 150-169 | 142-155 | 125-139 | 247-328 | 300-450 |
| GABA (µg/24h) | 4-4.8 | 3.9-4.9 | 2.5-3.5 | 3.5-4.5 | 12-19 | 16-30 |

The results obtained in urine tests revealed the efficacy of the transdermal patches for auriculotherapy of the present invention. Thus, the groups that were treated with the patches of chocolate and glutamine for auriculotherapy (groups I to IV) all presented an increase in the serotonin levels and a decrease in the urine dopamine levels, as well as a decrease below 4 µg/24h of GABA levels. In addition, the levels of serotonin and dopamine in groups III and IV were slightly higher and slightly lower, respectively, for these groups, which were treated with transdermal patches for auriculotherapy by iontophoresis, as compared with the groups treated with transdermal patches without iontophoresis.

In addition, the recovery of all patients in groups I, II, III and IV was noted in the clinical consultation, and their signs and symptoms of anxiety had improved and experienced better sleep quality and duration. However, only two patients in group V perceived a slight improvement in their sleep, but none of the patients in control group VI experienced improvement with respect to their anxiety during treatment.

No side effects associated with the transdermal patches for auriculotherapy according to the present invention were shown in any of the groups and two patients of group V indicated discomfort by wearing a large transdermal patch visible on the arm.

## Claims

1. Transdermal patch for use in auriculotherapy comprising at least one active substance selected from caffeine, chocolate and glutamine for the modulation of neurotransmitters, wherein the transdermal patch total area ranges between 10 and 200 mm².

2. Patch, according to claim 1, characterized also in that it comprises at least one further ingredient or cofactor selected from the list comprising L-tyrosine, phenylalanine, 5-htp decarboxylase, 5-hydroxy-tryptophane, tryptophan, theobromine, anandamide, phenylethylamine, gamma hydroxybutyrate acid, gamma hydroxybutyric acid, GABA transaminase, theanine, taurine, homotaurine, melatonin, carnitine, acetyl-carnitine, S-Adenosylmethionine, N-acetyl L-cysteine, N-acetyl-transferase, phosphatidylserine, inositol, phosphatidylinositol, catalase, dopamine beta-hydroxylase, L-dopa descarboxylase, NADH, magnesium, zinc, manganese, chromium, copper, selenium, iron, calcium, lithium, calcium citrate, vitamin C, vitamin B, coenzyme Q10, omega-3, ashwagandha, Melissa oficinalis, Siberian ginseng, St. John's wort, valerian, Ginko biloba, Mucuna pruriens, turmeric, Rhodiola rosea and Bacopa monniera extracts, or a combination thereof.

3. Patch, according to any one of the preceding claims, **characterized in that** said active substances are present at a concentration between 1% by weight and 90% by weight, relative to the total of components.

4. Patch, according to any one of the preceding claims, **characterized in that** said active substances are present at an amount ranging from 0.01 and 1000 mg.

5. Patch, according to claim 4, **characterized in that** said active substances are present at an amount ranging from 0.1 and 750 mg.

6. Patch, according to any of claims 2 to 5, **characterized in that** said further ingredient or cofactor is present at a concentration ranging between 1% by weight and 90% by weight relative to the total of components.

7. Patch, according to any of claims 2 to 5, **characterized in that** said further ingredient or cofactor is present at a concentration ranging between 0.01 and 1000 mg.

8. Patch, according to any one of the preceding claims, **characterized in that** it comprises the following structural elements:
- a deposit containing at least one active substance,
- a release controlling system,
- an adhesive surface for fixing the patch to the skin, and/or
- a film or protective covering.

9. Patch, according to claim 8, **characterized in that** said adhesive surface acts likewise as a deposit containing the at least one active substances.

10. Patch according to any of the preceding claims, **characterized in that** it comprises
- an outer layer of tissue containing alternating copper and zinc voltaic cells,
- an adhesive layer or surface containing the at least one active substance,
and it is applied by passive iontophoresis.

11. Patch, according to claim 1, **characterized in that** said total area ranges between 50 and 100 mm².

12. Patch, according to any one of claims 1 through 11, for use in the treatment of physiological, neurological and neurohormonal disorders.

13. Patch, for use according to claim 12, **characterized in that** said physiological, neurological and neurohormonal disorder is selected from depression or depressive mood, eating disorders such as bulimia and anorexia, disorders involving addiction to toxic substances, mental disorders such as schizophrenia, neurological diseases such as Parkinson's, appetite regulation and overweight control, increase of libido and regulation of prolactin levels, regulation of motor activity and cognitive functions, of learning and memory, for treating attention deficits, for the treatment of pain as anaesthetic, to control nausea and vomiting and even to improve immunity, for insomnia, to control panic attacks, stress, aggressiveness, tobacco use, headaches and migraines, for regulating circadian rhythm, gastrointestinal movement and neuroendocrine functions, for the treatment of irritable bowel, pain in chronic cases such as fibromyalgia or post-traumatic stress.

14. Patch, for use according to claim 12 or 13 **characterized in that** said treatment lasts between 2 weeks and 18 months.

## Patentansprüche

1. Transdermales Pflaster zur Verwendung in der Aurikulotherapie, umfassend mindestens einen Wirkstoff, ausgewählt aus Koffein, Schokolade und Glutamin, zur Modulation von Neurotransmittern, wobei die Gesamtfläche des transdermalen Pflasters zwischen 10 und 200 mm² liegt.

2. Pflaster nach Anspruch 1, **dadurch gekennzeichnet, dass** es mindestens einen weiteren Bestandteil oder Kofaktor umfasst, der aus der Liste ausgewählt ist, die umfasst: L-Tyrosin, Phenylalanin, 5-HTP-Decarboxylase, 5-Hydroxytryptophan, Tryptophan, Theobromin, Anandamid, Phenylethylamin, Gamma-Hydroxybutyrat, Gamma-Hydroxybuttersäure, GABA-Transaminase, Theanin, Taurin, Homotaurin, Melatonin, Carnitin, Acetylcarnitin, S-Adenosylmethionin, N-Acetyl-L-Cystein, N-Acetyltransferase, Phosphatidylserin, Inositol, Phosphatidylinositol, Katalase, Dopamin-Beta-Hydroxylase, L-Dopa-Descarboxylase, NADH, Magnesium, Zink, Mangan, Chrom, Kupfer, Selen, Eisen, Kalzium, Lithium, Kalziumcitrat, Vitamin C, Vitamin B, Coenzym Q10, Omega-3, Ashwagandha, Melissa officinalis, Sibirischer Ginseng, Johanniskraut, Baldrian, Ginko biloba, Mucuna pruriens, Kurkuma, Rhodiola rosea und Bacopa monniera-Extrakte oder eine Kombination davon.

3. Pflaster nach einem der vorstehenden Ansprüche, **dadurch gekennzeichnet, dass** die Wirkstoffe in einer Konzentration zwischen 1 Gew.-% und 90 Gew.-% bezogen auf die Gesamtmenge der Komponenten vorliegen.

4. Pflaster nach einem der vorstehenden Ansprüche, **dadurch gekennzeichnet, dass** die Wirkstoffe in einer Menge im Bereich von 0,01 bis 1000 mg vorliegen.

5. Pflaster nach Anspruch 4, **dadurch gekennzeichnet, dass** die Wirkstoffe in einer Menge im Bereich von 0,1 bis 750 mg vorliegen.

6. Pflaster nach einem der Ansprüche 2 bis 5, **dadurch gekennzeichnet, dass** der weitere Bestandteil oder Kofaktor in einer Konzentration im Bereich zwischen 1 Gew.-% und 90 Gew.-% bezogen auf die Gesamtmenge der Komponenten vorliegt.

7. Pflaster nach einem der Ansprüche 2 bis 5, **dadurch gekennzeichnet, dass** der weitere Bestandteil oder Kofaktor in einer Konzentration im Bereich zwischen 0,01 und 1000 mg vorhanden ist.

8. Pflaster nach einem der vorstehenden Ansprüche, **dadurch gekennzeichnet, dass** es die folgenden Strukturelemente umfasst:
- eine Ablagerung, die mindestens einen Wirkstoff enthält,
- ein Freisetzungskontrollsystem,
- eine Klebefläche zur Befestigung des Pflasters auf der Haut und/oder
- eine Folie oder Schutzabdeckung.

9. Pflaster nach Anspruch 8, **dadurch gekennzeichnet, dass** die Klebefläche ebenfalls als Depot für den mindestens einen Wirkstoff dient.

10. Pflaster nach einem der vorstehenden Ansprüche, **dadurch gekennzeichnet, dass** es
- eine äußere Gewebeschicht mit abwechselnden Kupfer- und Zink-Voltakammern,
- eine Klebeschicht oder -fläche, die den mindestens einen Wirkstoff enthält, umfasst und es durch passive lontophorese aufgetragen wird.

11. Pflaster nach Anspruch 1, **dadurch gekennzeichnet, dass** die Gesamtfläche zwischen 50 und 100 mm² liegt.

12. Pflaster nach einem der Ansprüche 1 bis 11 zur Verwendung bei der Behandlung von physiologischen, neurologischen und neurohormonellen Störungen.

13. Pflaster zur Verwendung nach Anspruch 12, **dadurch gekennzeichnet, dass** die physiologische, neurologische und neurohormonale Störung ausgewählt ist aus Depression oder depressiver Verstimmung, Essstörungen wie Bulimie und Anorexie, Störungen, die eine Abhängigkeit von toxischen Substanzen beinhalten, psychischen Störungen wie Schizophrenie, neurologischen Erkrankungen wie Parkinson, Appetitregulierung und Kontrolle von Übergewicht, Steigerung der Libido und Regulierung des Prolaktinspiegels, Regulierung der motorischen Aktivität und kognitiven Funktionen, des Lernens und des Gedächtnisses, zur Behandlung von Aufmerksamkeitsdefiziten, zur Schmerzbehandlung als Betäubungsmittel, zur Kontrolle von Übelkeit und Erbrechen und sogar zur Verbesserung der Immunität, bei Schlaflosigkeit, zur Kontrolle von Panikattacken, Stress, Aggressivität, Tabakkonsum, Kopfschmerzen und Migräne, zur Regulierung des zirkadianen Rhythmus, der gastrointestinalen Bewegung und der neuroendokrinen Funktionen, zur Behandlung von Reizdarm, Schmerzen in chronischen Fällen wie Fibromyalgie oder posttraumatischem Stress.

14. Pflaster zur Verwendung gemäß Anspruch 12 oder 13, **dadurch gekennzeichnet, dass** die Behandlung zwischen 2 Wochen und 18 Monaten dauert.

## Revendications

1. Timbre transdermique pour l'auriculothérapie comprenant au moins un principe actif choisi parmi la caféine, le chocolat et la glutamine pour la modulation des neurotransmetteurs, dans lequel la surface totale de timbre transdermique est comprise entre 10 et 200 mm².

2. Timbre, selon la revendication 1, caractérisé également en ce qu'il comprend au moins un autre ingrédient ou cofacteur choisi dans la liste comprenant la L-tyrosine, la phénylalanine, la 5-htp décarboxylase, le 5-hydroxy-tryptophane, le tryptophane, la théobromine, l'anandamide, la phényléthylamine, l'acide gamma-hydroxybutyrate, l'acide gamma-hydroxybutyrique, la GABA transaminase, la théanine, la taurine, l'homotaurine, la mélatonine, la carnitine, l'acétyl-carnitine, la S-Adénosylméthionine, le N-acétyl L-cystéine, le N-acétyl-transférase, le phosphatidylsérine, l'inositol, le phosphatidylinositol, la catalase, la dopamine bêta-hydroxylase, la L-dopa descarboxylase, le NADH, le magnésium, le zinc, le manganèse, le chrome, le cuivre, le sélénium, le fer, le calcium, le lithium, le citrate de calcium, la vitamine C, la vitamine B, la coenzyme Q10, les oméga-3, l'ashwagandha, la Melissa oficinalis, le ginseng de Sibérie, le millepertuis, la valériane, le Ginko biloba, le Mucuna pruriens, le curcuma, les extraits de Rhodiola rosea et de Bacopa monniera, ou une combinaison de ces éléments.

3. Timbre, selon l'une quelconque des revendications précédentes, **caractérisé en ce que** lesdits principes actifs sont présents à une concentration comprise entre 1% en poids et 90% en poids, par rapport au total des composants.

4. Timbre, selon l'une quelconque des revendications précédentes, **caractérisé en ce que** lesdits principes actifs sont présents à une quantité comprise entre 0,01 et 1000 mg.

5. Timbre, selon la revendication 4, **caractérisé en ce que** lesdits principes actifs sont présents à une quantité comprise entre 0,1 et 750 mg.

6. Timbre, selon l'une quelconque des revendications 2 à 5, **caractérisé en ce que** ledit autre ingrédient ou cofacteur est présent à une concentration comprise entre 1 % en poids et 90 % en poids par rapport au total des composants.

7. Timbre, selon l'une quelconque des revendications 2 à 5, **caractérisé en ce que** ledit autre ingrédient ou cofacteur est présent à une concentration comprise entre 0,01 et 1000 mg.

8. Timbre, selon l'une quelconque des revendications précédentes, **caractérisé en ce qu'**il comprend les éléments structurels suivants :
- un dépôt contenant au moins un principe actif,
- un système de contrôle de libération,
- une surface adhésive pour fixer le timbre sur la peau, et/ou
- un film ou un revêtement protecteur.

9. Timbre selon la revendication 8, **caractérisé en ce que** ladite surface adhésive agit également comme un dépôt contenant ledit au moins un principe actif.

10. Timbre selon l'une quelconque des revendications précédentes, **caractérisé en ce qu'**il comprend
- une couche externe de tissu contenant des cellules voltaïques de cuivre et de zinc en alternance,
- une couche ou une surface adhésive contenant au moins un principe actif, et il est appliqué par une iontophorèse passive.

11. Timbre, selon la revendication 1, **caractérisé en ce que** ladite surface totale est comprise entre 50 et 100 mm².

12. Timbre, selon l'une quelconque des revendications 1 à 11, pour utilisation dans le traitement des troubles physiologiques, neurologiques et neurohormonaux.

13. Timbre, pour utilisation selon la revendication 12 **caractérisé en ce que** ledit trouble physiologique, neurologique et neurohormonal est choisi parmi la dépression ou l'humeur dépressive, les troubles alimentaires tels que la boulimie et l'anorexie, les troubles impliquant une dépendance à des substances toxiques, les troubles mentaux tels que la schizophrénie, les maladies neurologiques telles que la maladie de Parkinson, la régulation de l'appétit et le contrôle du surpoids, l'augmentation de la libido et la régulation des niveaux de prolactine, la régulation de l'activité motrice et des fonctions cognitives, de l'apprentissage et de la mémoire, pour traiter les déficits d'attention, pour le traitement de la douleur comme anesthésiant, pour contrôler les nausées et les vomissements et même pour améliorer l'immunité, pour l'insomnie, pour contrôler les attaques de panique, le stress, l'agressivité, le tabagisme, les maux de tête et les migraines, pour réguler le rythme circadien, les mouvements gastro-intestinaux et les fonctions neuroendocriniennes, pour le traitement de l'intestin irritable, de la douleur dans les cas chroniques comme la fibromyalgie ou le stress post-traumatique.

14. Timbre, pour utilisation selon la revendication 12 ou 13, **caractérisé en ce que** ledit traitement dure entre 2 semaines et 18 mois.
